(19) Europäisches Patentamt
European Patent Office
Office européen des brevets

(11) EP 3 581 553 B1

(12) **EUROPEAN PATENT SPECIFICATION**

(45) Date of publication and mention of the grant of the patent:
**14.10.2020 Bulletin 2020/42**

(51) Int Cl.:
***C07C 31/42** (2006.01)* ***B01D 17/02** (2006.01)*
***C09K 3/32** (2006.01)*

(21) Application number: **18305732.2**

(22) Date of filing: **15.06.2018**

(54) **NOVEL FLUORINATED POLYOLS AND THEIR USE AS ORGANOGELATORS**

NEUARTIGE FLUORIERTE POLYOLE UND DEREN VERWENDUNG ALS ORGANOGELATOREN

NOUVEAUX POLYOLS FLUORÉS ET LEUR UTILISATION COMME DES ORGANOGÉLATEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**18.12.2019 Bulletin 2019/51**

(73) Proprietors:
- **CENTRE NATIONAL DE LA RECHERCHE SCIENTIFIQUE**
  **75016 Paris (FR)**
- **Universite d'Aix-Marseille**
  **13007 Marseille (FR)**

(72) Inventors:
- **QUINTARD, Adrien**
  **13013 Marseille (FR)**
- **SPERANDIO, Céline**
  **42000 Saint-Etienne (FR)**
- **RODRIGUEZ, Jean**
  **13016 Marseille (FR)**

(74) Representative: **Cabinet Becker et Associés**
**25, rue Louis le Grand**
**75002 Paris (FR)**

(56) References cited:
**US-A1- 2008 113 573** **US-A1- 2012 108 680**

- **TERECH P ET AL: "LOW MOLECULAR MASS GELATORS OF ORGANIC LIQUIDS AND THE PROPERTIES OF THEIR GELS", CHEMICAL REVIEWS, AMERICAN CHEMICAL SOCIETY, US, vol. 97, 1 January 1997 (1997-01-01), pages 3133-3159, XP002127859, ISSN: 0009-2665, DOI: 10.1021/CR9700282**

EP 3 581 553 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The invention relates to new fluorinated polyols and their use as organogelators. It also relates to organogels obtained by contacting such compounds with a liquid sample comprising at least one oil and/or at least one organic solvent, and uses thereof.

### TECHNICAL BACKGROUND

Gelation is based on the ability of certain molecules to be organized in a macro- or supramolecular network in a liquid. During the process, the liquid ends up trapped by the network and the resulting solid gel, composed of a liquid which cannot flow, is obtained. Two types of gelation, depending on the nature of the molecular network, are commonly described: chemical gelation and physical gelation. Chemical gelation corresponds to the formation of a network through a chemical reaction and molecules are therefore associated by strong bonds, such as covalent bonds. The resulting gels are highly stable and their formation is irreversible. This chemical gelation is typically observed in biological processes, such as blood clotting or wound healing. Physical gelation corresponds to the formation of a network, whose molecules are associated by weak interactions, such as Van der Waals type interactions and hydrogen bonds. Unlike chemical gelation, physical gelation is a reversible process, which may depend on several physico-chemical parameters (concentration, temperature, solvent, pH...). Therefore, conditions may be adapted for stabilizing the physical gel or returning to a liquid state.

More particularly, organogelators are small molecules which display an ability to form a physical gel, when contacting an organic solvent, a mineral or organic oil, at very low concentrations. These small molecules self-assemble in a high order through numerous weak interactions, and the resulting supramolecular structure may have various morphologies: fibers, cylinders, helixes, plates. Due to their properties, organogelators or gels resulting therefrom can be found in many fields: cosmetics, food, healthcare, personal care, paints, lubricants and catalysis. A particular application is the removal of oil, typically after an oil spill: oil compounds act as the liquid phase and addition of organogelators triggers the formation of a solid gel, which can be easily removed from the polluted water.

For example, CN 104892417 discloses novel organogelators of formula (Ia),

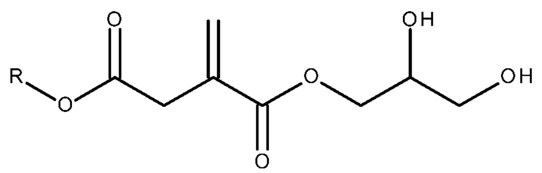

(Ia),

wherein R is an alkyl chain having at least 10 carbon atoms, which bears polar hydroxy groups and a long hydrophobic chain. These compounds, which self-assemble, have high adsorption capacities, particularly towards organic solvents, and are particularly well-suited for purification of waste water or treatment of oil spill.

US 2012/0108680 describes long-chain glycyl polyol type gelators and gels of formula (Ib),

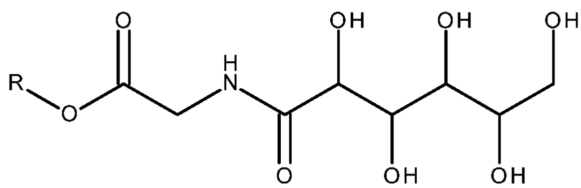

(Ib),

wherein R is a $C_{18-20}$ aliphatic chain. These compounds are composed of a hydrophilic moiety, namely the glycine and polyol groups, and a hydrophobic moiety, namely the long aliphatic chain. The general structure allows the compound to self-assemble in a tubular or plate-like shape. Said compounds display a high environmental compatibility, biocompatibility and biodegradability and therefore, can be used in cosmetics, food, or for pharmaceutical or medical applications.

US 2008/0113172 and US 2008/0113573 describe fluorinated amino-acid derivatives, which self-assemble in solution, displaying thus a gelator behavior. Urea-amide gelators containing a perfluorinated chain are also described in an article by Raghavanpillai and Franco (Appl. Sci. 2012, 2, 175-191). These kinds of gelators are particularly interesting

for surface treatment or modification, since the structure of such compounds provides oil- and water-repellency properties to substrates, and enables to reach low surface energies. It is suggested that the fluorinated groups play a prominent role in the oleophobic behavior and could further lower said surface energy.

An article by Quintard and Rodriguez (ACS Catal. 2017, 7, 5513-5517) describes a method for the stereoselective synthesis of fluorinated keto-diols through a multi-catalyzed three-component cascade transformation. It is shown that a two-steps process consisting of a reduction by sodium borohydride followed by acetalization of the keto diol leads to a protected fluorinated triol with good stereoselectivities. Several keto-diols were prepared with various substituting hydrocarbon groups.

## SUMMARY OF THE INVENTION

The Applicant has demonstrated that similar but unprotected triols displayed an organogelator behavior, in particular when these triols were substituted with long aliphatic chains. Furthermore, it was observed, in particular by X-ray structure analysis, that fluorine groups, associated with the triol moiety, allowed a rigidification of the structure and had a strong ability to create hydrogen-bonding networks, which accounts for their crucial role in the organogelator behavior of these compounds. While concentrations of at least 5 % are usually required to obtain an organogel with organogelators of the above prior art, Critical Gel Concentrations (CGC) of less than or equal to 2 % were obtained in a wide diversity of solvents and/or oils with the compounds of the invention.

Hence, the invention relates to a compound represented by formula (I):

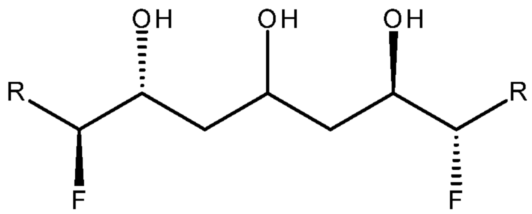

(I),

wherein R is a hydrocarbon aliphatic chain having from 6 to 26 carbon atoms, and/or its enantiomer.

It also relates to a use of a compound as defined above, as an organogelator compound in a liquid sample comprising at least one oil and/or at least one organic solvent.

It further relates to an organogel obtained by contacting a compound as defined above, with a liquid sample comprising at least one oil and/or at least one organic solvent.

Another object of the present invention is the use of an organogel as defined above for anion sensing or as a catalyst or in a catalytic system.

Another object of the present invention is a composition comprising at least one compound as defined above, or at least one organogel as defined above.

The present invention also relates to a process for separating a hydrophobic phase from a mixture of hydrophobic and hydrophilic phases, comprising the steps of:

a) contacting at least one compound as defined above with a mixture of hydrophobic and hydrophilic phases to obtain an organogel comprising the hydrophobic phase and a residue comprising the hydrophilic phase;
b) separating said organogel from said residue;

and optionally :

c) heating said organogel to a temperature allowing the deconstruction of said organogel into a hydrophobic phase and said at least one compound;
d) separating said hydrophobic phase and said at least one compound.

## DETAILED DESCRIPTION OF THE INVENTION

In the following description, the expression "comprised between" is intended to include the upper and lower limits within the range described.

The present invention provides a compound represented by formula (I):

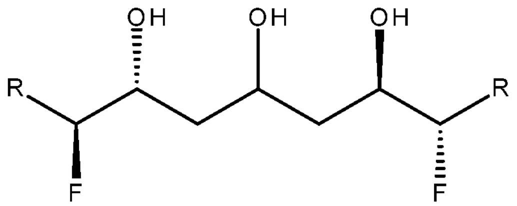

(I),

wherein R is a hydrocarbon aliphatic chain having from 6 to 26 carbon atoms, and/or its enantiomer.

The expression "hydrocarbon aliphatic chain" refers to a linear or branched, cyclic or acyclic, non-aromatic hydrocarbon chain, optionally substituted, and optionally comprising at least one double bond and/or at least one triple bond. Examples of substituents include, but are not limited to, NO2, CN, F, Cl, Br, I, CF3, a $C_{1-12}$ alkyl, a $C_{2-12}$ alkenyl, a $C_{2-12}$ alkynyl, a $C_{3-12}$ cycloalkyl, a $C_{1-12}$ alkoxy, $C_{1-12}$ alkylamino or $C_{1-12}$ dialkylamino.

"$C_{1-12}$ alkyl" refers to a linear or branched, acyclic, saturated hydrocarbon chain having 1 to 12 carbon atoms. Examples of $C_{1-12}$ alkyl include, but are not limited to, methyl, ethyl, propyl or dodecyl.

"$C_{2-12}$ alkenyl" refers to a linear or branched, acyclic, unsaturated hydrocarbon chain having at least one carbon-carbon double bond and having 2 to 12 carbon atoms. Examples of $C_{2-12}$ alkenyl include, but are not limited to, ethenyl, butenyl or prenyl.

"$C_{2-12}$ alkynyl" refers to a linear or branched, acyclic, unsaturated hydrocarbon chain having at least one carbon-carbon triple bond and having 2 to 12 carbon atoms. Examples of $C_{2-12}$ alkynyl include, but are not limited to, ethynyl or propargyl.

"$C_{3-12}$ cycloalkyl" refers to a mono- or polycyclic saturated hydrocarbon chain having 3 to 12 carbon atoms. Examples of $C_{3-12}$ cycloalkyl include, but are not limited to, cyclopropyl, cyclopentyl or cyclohexyl.

"$C_{1-12}$ alkoxy" refers to a $C_{1-12}$ alkyl as defined above attached to the molecule by a -O- (ether) bond, i.e. a ($C_{1-12}$ alkyl)-O- group. Examples of $C_{1-12}$ alkoxy include, but are not limited to, methoxy or ethoxy.

"$C_{1-12}$ alkylamino" refers to a ($C_{1-12}$ alkyl)-NH- group, wherein $C_{1-12}$ alkyl is as defined above. An example of $C_{1-12}$ alkylamino includes, but is not limited to, methylamino.

"$C_{1-12}$ dialkylamino" refers to a ($C_{1-12}$ alkyl)$_2$N- group, wherein $C_{1-12}$ alkyl is as defined above. An example of $C_{1-12}$ dialkylamino includes, but is not limited to, dimethylamino.

Examples of hydrocarbon aliphatic chains having from 6 to 26 carbon atoms include, but are not limited to, hexyl, heptyl, octyl, nonyl, decyl, dodecyl, tetradecyl or any fatty chain found in vegetable or animal oils such as a stearyl, oleyl, linoleyl, erucyl or palmitoleyl group.

In a preferred embodiment, R is a hydrocarbon aliphatic chain having from 8 to 14 carbon atoms. More preferably, R is an octyl group.

It is understood that the invention pertains to a compound represented by formula (I) and/or its enantiomer, in other words, both enantiomers may be considered separately or as a racemate.

Also, it is understood that "one enantiomer" may refer to a compound existing as one enantiomer, or to a mixture of this compound with small amounts of its enantiomer, such that the enantiomeric excess of said mixture is above 98 %, preferably above 99 %.

The enantiomeric excess "ee" is defined by the following equation (1):

$$ee = |X_+ - X_-| \times 100\ \%, \qquad (1)$$

wherein X+ and X- are mole fractions of enantiomer (+) and enantiomer (-) respectively, with ($X_+$ + X-) = 1.

The enantiomeric purity p+ of enantiomer (+) is defined by the following equation (2):

$$p_+ = X_+ \times 100\ \%, \qquad (2)$$

wherein X+ is as defined above.

The enantiomeric purity p- of enantiomer (-) is defined by the following equation (3):

$$p_- = X_- \times 100\ \%, \qquad (3)$$

wherein X- is as defined above.

The diastereomeric ratio is the ratio of the mole fraction of one diastereomer in a mixture to the mole fraction

of the other diastereomer(s).

A compound according to the invention may be prepared according to a process comprising the following steps:

a) reacting 1,3-acetonedicarboxylic acid with a fluorinating agent and an aldehyde of formula R-CH2-CHO in the presence of a chiral organocatalyst and a metal catalyst, in order to form a compound of formula (II):

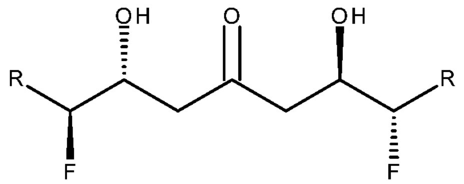

(II),

wherein R is as defined in formula (I), and/or its enantiomer;
b) reacting the compound obtained in step (a) with a reducing agent.

Conditions (such as temperature, concentration, solvents or equivalents of the reactants) described below for each step may be adjusted by the skilled artisan using his/her general background. Each intermediate or product obtained at the end of a step may be isolated and optionally purified. Alternatively, several steps may be carried out one-pot without isolating said intermediate or product.

In step (a) of the process described above, the fluorinating agent may be any fluorinated compound whose fluor(s) can be released or transferred to another compound, such as NFSI (N- fluorobenzenesulfonimide) or F-TEDA (N-Chloromethyl-N-fluorotriethylenediammonium bis(tetrafluoroborate)). In a preferred embodiment, the fluorinating agent is NFSI. The reaction of step (a) is catalyzed by two catalysts: a chiral organocatalyst and a metal catalyst. The metal catalyst is advantageously an organometallic complex, preferably comprising a transition metal such as Cu or Fe, such as Fe(acac)3, Cu(tmhd)2, Cu(hfac)2 or Cu(acac)2. Preferably the metal catalyst is Cu(acac)2. The chiral organocatalyst is a chiral non-metallic organic catalyst.

Examples of chiral organocatalysts include, but are not limited to, a catalyst of formula (III) or (IV), or salts thereof:

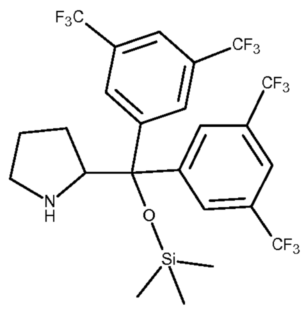

(III),

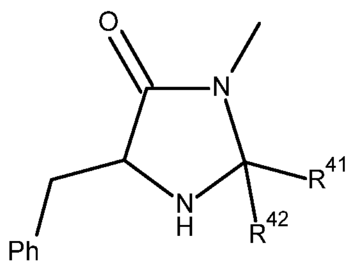

(IV),

wherein $R^{41}$ and $R^{42}$ represent independently a radical selected from the group consisting of a hydrogen atom and a $C_{1-12}$ alkyl. In one embodiment, $R^{41}$ is a methyl and $R^{42}$ is a methyl. In another embodiment, $R^{41}$ is a tert-butyl and $R^{42}$ is a hydrogen atom.

The enantiomer of a compound of formula (II) obtained with enantiomer ***(S)*** of said organocatalyst may be prepared by using the corresponding enantiomer ***(R)*** of said organocatalyst. A racemic mixture comprising a compound of formula (II) and its enantiomer may be obtained by using a racemic mixture of said chiral organocatalyst.

Preferably, the chiral organocatalyst exists as one single enantiomer (R or S).
More preferably, the chiral organocatalyst is one enantiomer (R or S) of the catalyst represented by formula (III), as represented above.

In step (a), the metal catalyst and the chiral organocatalyst may be added simultaneously or successively. In a preferred embodiment, the metal catalyst is added between 30 minutes and 2 hours after the chiral organocatalyst.
The amount of the fluorinating agent may be comprised between 2 and 6 equivalents, preferably between 3 and 5 equivalents, relative to the amount of 1,3-acetonedicarboxylic acid. The amount of the aldehyde R-CH2-CHO may be comprised between 2 and 6 equivalents, preferably between 3 and 5 equivalents, relative to the amount of 1,3-acetonedicarboxylic acid. The compound obtained in step (a) is a compound of formula (II) and/or its enantiomer. When the chiral organocatalyst exists as one enantiomer, the enantiomeric excess of the reaction of step (a) is advantageously

greater than or equal to 95 %, preferably greater than or equal to 98 %, as measured by chiral Gas Chromatography (GC) or Mosher ester analysis. A purification (or resolution in case of a racemate), such as crystallization or chiral chromatography, may be carried out in order to separate and isolate one compound from its enantiomer.

In step (b) of the process described above, the ketone function of the compound obtained in step (a), and/or its enantiomer, is reduced into an alcohol by a reducing agent, such as sodium borohydride, lithium aluminum hydride, or any other hydride source, preferably sodium borohydride. The amount of the reducing agent may be comprised between 1 and 4 equivalents, preferably between 1.1 and 2 equivalents, relative to the amount of the compound obtained in step (a). Step (b) leads to a compound of formula (I) and/or its enantiomer. A purification (recrystallization, chromatography...) may be further carried out in order to eliminate all or part of one or more diastereomers of said compound of formula (I) and/or of its enantiomer.

The diastereomeric ratio of the compound obtained at step (b) may be greater than or equal to 98:2, preferably greater than or equal to 99:1, as measured by $^{19}F$ NMR spectroscopy, Mosher ester analysis, or any other technique known by the skilled artisan.

Use as organogelator

Such a compound represented by formula (I) and/or its enantiomer, is particularly well-suited to be used as an organogelator. Contacting said organogelator with a liquid sample, typically comprising at least one oil and/or at least one organic solvent, may result in the formation of an organogel.

The term "organogelator" refers to a compound having an ability to self-assemble, in a liquid sample, into a three-dimensional nano-network of self-assembled structures, such as fibers. The self-assembly may typically result from a plurality of intermolecular non-covalent interactions, such as hydrogen bonding, van der Waals, pi-stacking, electrostatic or charge-transfer interactions. The terms "gelator" or "gelling agent" may be alternatively used instead of "organogelator".

The term "organogelator" may be used in the present application to refer to "one or more compounds of the invention used as organogelator".

The "organogelator" may refer to one or more compounds of formula (I) and/or one or more enantiomers of compounds of formula (I). In particular, it may be a racemate, i.e. an equimolar mixture of a compound of formula (I) and its enantiomer. Preferably, the "organogelator" is one compound of formula (I) or its enantiomer.

When considered as one compound of formula (I) or its enantiomer, and not as a racemate, the enantiomeric purity of the organogelator, may be greater than or equal to 95 %, preferably greater than or equal to 98 %, as measured by chiral GC or Mosher ester analysis. The diastereomeric purity of a compound according to the invention used as an organogelator may be greater than or equal to 98 %, preferably greater than or equal to 99 %, as measured by $^{19}F$ NMR spectroscopy.

The term "organogel" (or alternatively "gel") refers to the solid or semi-solid material comprising a mixture of said self-assembled structures such as fibers, constituted of at least one compound of the invention, with all or part of said liquid sample, entrapped in pores between self-assembled structures.

Said at least one oil may be a mineral, animal, vegetable oil, or a mixture thereof. Examples of mineral oils include, but are not limited to, oils from petroleum, such as petroleum fractions (diesel, Cracked Run Naphta, Straight Run Naphta, etc.), higher order alkanes, or fuel oil, or from coal, a silicone oil, liquid paraffin, or mixtures thereof. More broadly, a mineral oil may refer to crude oil. Examples of vegetable oils include, but are not limited to, argan oil, canola oil, jojoba oil, sweet almond oil or a mixture thereof. Examples of animal oils include, but are not limited to, fish oil.

Examples of organic solvents include, but are not limited to, aliphatic hydrocarbons such as pentane or hexane, alicyclic hydrocarbons such as cyclohexane, aromatic hydrocarbons such as benzene, styrene, toluene, *ortho*-xylene, meta-xylene or *para*-xylene, halogenated hydrocarbons such as dichloromethane, chloroform or chlorobenzene, nitrogen-based solvents such as acetonitrile or triethylamine, oxygen-based solvents, in particular ketones such as acetone, ethers such as diethyl ether, methyl tert-butyl ether or tetrahydrofurane (THF) and alcohols such methanol or ethanol, esters or amides, such ethyl acetate or dimethylformamide (DMF), and mixtures thereof.

In one particular embodiment, the liquid sample comprises at least one oil and/or at least one organic solvent, and further comprises water. The liquid sample may thus be composed of a hydrophilic phase and a hydrophobic phase. The hydrophobic phase may typically comprise said at least one oil and/or said at least one organic solvent. The hydrophilic phase may typically comprise water. The hydrophilic phase and the hydrophobic phase are advantageously poorly miscible or not miscible. Both phases may also form a water-in-oil emulsion. The compound of the invention, used as an organogelator, is preferably soluble in the hydrophobic phase and poorly soluble or not soluble in the hydrophilic phase.

Contacting an organogelator with a liquid sample may result in the formation of an organogel. Said organogel may be formed when the concentration of said compound is greater than or equal to the critical gel concentration (CGC) and when the temperature of the liquid sample, comprising said organogelator, is less than or equal to the gel transition

temperature (Tg). The gel transition temperature (Tg) is defined as the threshold temperature for the deconstruction of an organogel.

The critical gel concentration of the organogelator of this invention is advantageously less or equal to 5 % (w/v), preferably less or equal to 2 % (w/v), even more preferably less or equal to 1 % (w/v), when measured at 25 °C. Volume V that is considered for assessing the concentration is the volume in which said compound is soluble, i.e. the volume of the entire liquid sample or of a part of the liquid sample such as the hydrophobic phase.

The formation of said organogel may be triggered by a cooling step, optionally preceded by a heating step. In one embodiment, the temperature of the liquid sample comprising said organogelator is above the gelation temperature. In this embodiment, the formation of said organogel may be triggered by a cooling step, from a temperature above the gelation temperature to a temperature below the gelation temperature. In another embodiment, the temperature of the liquid sample comprising said organogelator is under the gelation temperature. In this embodiment, the formation of said organogel may be triggered by a heating step followed by a cooling step. The heating step may be carried out from a temperature below the gelation temperature to a temperature above the gelation temperature. The cooling step may be carried out from a temperature above the gelation temperature to a temperature below the gelation temperature. In yet another embodiment, the temperature of the liquid sample comprising said organogelator may be under the gelation temperature, and said organogel may be formed by a heating step at a temperature below the gelation temperature. Alternatively, the temperature of the liquid sample comprising said organogelator may be under the gelation temperature, and said organogel may be formed without a heating step.

The formation of said organogel may be modulated, i.e. triggered, facilitated or slowed down, by adjusting temperature, pressure and/or pH, and/or by adding a chemical compound, such as a salt or a Bronsted or Lewis acid, and/or by sonicating.

In one particular embodiment, the compound of the invention is used as an organogelator, for treating an oil spill. In this embodiment, the liquid sample may thus be a sample of lake, sea, ocean or river water contaminated with spilled oil. Spilled oil may comprise crude oil or, more broadly, any oil or oil mixture from petroleum, such as petroleum fractions or fuel oil. Said liquid sample may therefore be composed of a hydrophilic phase comprising said lake, sea, ocean or river water and of a hydrophobic phase comprising spilled oil.

One object of the invention is a process for separating a hydrophobic phase from a mixture of hydrophobic and hydrophilic phases, comprising the steps of:

a) contacting at least one compound as defined above with a mixture of hydrophobic and hydrophilic phases to obtain an organogel comprising the hydrophobic phase and a residue comprising the hydrophilic phase;
b) separating said organogel from said residue;

and optionally:

c) heating said organogel to a temperature allowing the deconstruction of said organogel into a hydrophobic phase and said at least one compound as defined above;
d) separating said hydrophobic phase and said at least one compound as defined above.

In step (a), said at least one compound of the invention may be solubilized in (an) organic solvent(s) prior to be contacted with the mixture of hydrophobic and hydrophilic phases. The solution of said at least one compound in (an) organic solvent(s) may be heated to a temperature advantageously comprised between 20 °C and 130 °C, or may not be heated, prior to be contacted with the mixture of hydrophobic and hydrophilic phases. Step (a) consists of forming an organogel composed of said at least one compound entrapping selectively all or part of said hydrophobic phase, and thus entrapping low or zero quantities of said hydrophilic phase. The residue typically corresponds to the part of the mixture that is not entrapped in the organogel. The residue may therefore comprise all or part of said hydrophilic phase.

Step (b) consists of separating the organogel formed in step (a) and said residue. This separation may be carried out by filtration or by direct removal of the organogel from the medium. The isolated organogel obtained in step (b) may optionally be deconstructed by a heating step at a temperature typically higher than the gel transition temperature of said organogel. Alternatively, the deconstruction of said organogel may be triggered by a chemical compound, such as a salt. The hydrophobic phase and said at least one compound of the invention constituting said organogel may be recovered by the deconstruction of step (c). The hydrophobic phase and said at least one compound may optionally be separated by filtration or distillation. Said at least one compound may be recycled in step (a) of the process of the invention.

In a particular embodiment, said hydrophobic phase comprises spilled oil, and said hydrophilic phase comprises water. Said water is preferably lake, sea, ocean or river water.

Another object of the invention is an organogel obtained by contacting a compound as defined above, with a liquid sample comprising at least one oil and/or at least one organic solvent.

Said organogel may be used for anion sensing. "Anion sensing" may refer to the detection and/or the identification and/or the quantification of one or more anions. Anion sensing may be achieved by contacting said organogel with one or more salts containing one or more anions such as chloride, fluoride, bromide, iodide, tetrafluoroborate, nitrite, nitrate, sulfite, sulfate, chlorate, perchlorate, $R'SO_3^-$ or $R'CO_2^-$ wherein R' is an alkyl such as $CH_3$, or a perfluoroalkyl such as $CF_3$ or $C_2F_5$. Contacting may result in the deconstruction of said organogel, which may depend on the concentration and/or the nature of the anions.

Said organogel may also be used as a catalyst or as an entity in a catalytic system. For instance, said organogel may be associated with a metal, such as a metal complex or metal nanoparticles, to form a catalytic system. Alternatively, said organogel may be used as an organocatalyst. Said organogel may be used as a template to access various metal nanoparticles.

The invention also relates to a composition comprising at least one compound of the invention, or at least one organogel as defined above. Said composition may be a lubricant, adhesive, molding, sealing, cosmetic composition or a coating composition such as a paint, a varnish or an ink.

The invention will also be described in further detail in the following examples, which are not intended to limit the scope of this invention, as defined by the attached claims.

## EXAMPLES

NMR spectra were recorded on a Bruker AC 300 (300 MHz) or a Bruker AC 400 (400 MHz) spectrometer. Chemical shifts are given in ppm, using as internal standards the residual $CHCl_3$ signal for $^1H$ NMR ($\delta$ = 7.26) and the deuterated solvent signal for $^{13}C$ NMR ($\delta$ = 77.0). Data for $^{13}C$ NMR are reported as follows: chemical shift (multiplicity). Data for $^1H$ NMR are reported as follows: chemical shift (multiplicity [s = singlet, d = doublet, t = triplet, q = quadruplet, m = multiplet, br = broad], coupling constants *J* in Hertz (Hz), integration). Anhydrous THF, toluene, dichloromethane were obtained from a Solvent Purification System M Braun SPS-800.

Thin-Layer Chromatography (TLC) were developed on silica Merck 60F254 and revealed under UV lamp ($\lambda$ = 254 nm) and with universal stain: p-Anisaldehyde (prepared with 30g of ice, 60 mL of EtOH, 5 mL of $H_2SO_4$, 5 mL of p-anisaldehyde and 0.5 mL of AcOH). Flash Chromatography was performed following the method of Still on 40 - 63 $\mu$m silica gel eluted with the specified eluent.

High resolution mass spectra (HRMS) were performed on a QStar Elite (Applied Biosystems SCIEX) spectrometer equipped with atmospheric pression ionization source (API) pneumaticly assisted. Samples were ionized by positive electrospray mode as follows: electrospray tension (ISV): 5500 V; opening tension (OR): 50 V; nebulization gas pression (air): 20 psi.

Chiral GC analysis were performed on a HP 4890 using 6 bar argon as vector. Column: 25m/0,25 mm. Chromatogram analyzed with ChromNav software.

Optical rotations were measured at 20 °C in $CHCl_3$ with a Anton Paar MCP 200 polarimeter with a 0.2 cm length.

Absolute and relative configuration were determined by analogy on fluorinated triols by X-Ray analysis of compound (ent-6a) represented below assuming the same transition states for the other substrates. This is in agreement with ACS Catal. 2017, 7, 5513.

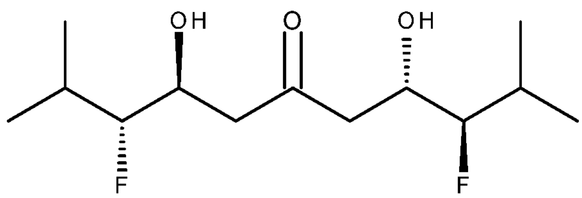

**(ent-6a)**

**Example 1:** Preparation of a compound of formula (I) (*"triol"*)

*a) First step: Synthesis of a compound of formula (II) ("keto-diol")*

Procedure A: The corresponding aldehyde R-CH2-CHO (32 mmol, 3.2 eq.) was dissolved in 40 mL of MTBE and cooled to 0°C under argon. 896.2 mg of **(*S*)-(III)** (1.5 mmol, 15 mol%) were added followed by addition over 1 minute of 9.10 g of NFSI (29 mmol, 2.9 eq.). The reaction mixture was then stirred at 0°C for 5 minutes then at room temperature for 1.5 hours. 1.62 g of 1,3-acetone-dicarboxylic acid (technical grade, 10 mmol, 1 eq.) and 392.4 mg of $Cu(acac)_2$ (1.5 mmol, 15 mol%) were simultaneously added and the reaction was stirred at room temperature for 4 hours more before addition of 40 mL of an aqueous solution saturated with $NH_4Cl$ and 20 mL of aqueous HCl 1M. The aqueous layer was extracted by 3 times 50 mL diethyl ether, the combined organic layers were washed by 25 mL saturated aqueous NaCl,

3 times 40 mL of saturated aqueous $NaHCO_3$, 25 mL of saturated aqueous NaCl, dried over $Na_2SO_4$, filtered and the solvent evaporated. Purification by recrystallization from a mixture of diethyl ether and n-hexane or dichloromethane and n-hexane directly provided the corresponding keto-diol of formula (II).

The enantiomer of a compound of formula (II) obtained with **(*S*)-(III)** catalyst may be prepared by using the enantiomer **(*R*)-(III).**

Compound **2a**

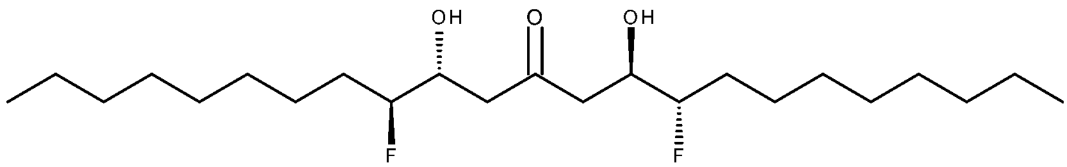

Compound **2a** was prepared according to procedure (A) using 3.8 mL g of decanal (20 mmol, 4 eq.), 435.1 mg of **(S)-(III)** (0.75 mmol, 15 mol%), 4.56 g ofNFSI (14.5 mmol, 2.9 eq.), 736.9 mg of 1,3-acetone-dicarboxylic acid (technical grade, 5 mmol, 1 eq.) and 196.2 mg of $Cu(acac)_2$ (0.75 mmol, 15 mol%). Purification over silica gel (petroleum ether/ethyl acetate (7/3)) yielded the keto-diol containing minor impurities. Recrystallization from a mixture of ethyl acetate and pentane gave the keto-diol **2a** with 20:1 dr.

882 mg (2,17 mmol). 43% yield. Rf = 0.6 (petroleum ether / ethyl acetate (7/3)).

$[\alpha]^{20}_D$ = +58.4° (THF, c = 1.1), 20:1 dr.

$^1$H NMR (400 MHz, THF-d8): $\delta$ (ppm) = 0.88 (t, 2 CH3), 1.28-1.40 (m, 12 CH2), 1.45-1.77 (m, 4 CH2), 2.55-2.68 (m, CH2), 4.00-4.07 (m, 2 CH), 4.14-4.18 (m, CH), 4.26-4.31 (m, CH). $^{13}$C NMR (75 MHz, THF-d8): $\delta$ (ppm) = 13.4 (s, CH3), 22.5 (s, CH2), 25.1 (d, J = 3 Hz, CH2), 29.2 (s, CH2), 29.4 (s, CH2), 30.8 (d, J = 20 Hz, CH2), 31.8 (s, CH2), 46.4 (d, J = 5 Hz, CH2), 68,5 (d, J = 23 Hz, CH(OH)), 95.7 (d, J = 170 Hz, CH(F)), 207.5 (CO).

$^{19}$F (NMR (400 MHz, THF-d8)): -190.7.

HRMS ESI $[M+Na]^+$ calculated for $C_{23}H_{45}F_2O^+$ : 407.3331. Observed: 407.3326.

*b) Second step: Reduction of the compound of formula (II)*

Procedure B: 1.65 mmol (1 eq.) of the ketodiol were dissolved in 7 mL of dry THF and 0.7 mL of MeOH and cooled to 0°C under argon. 81.0 mg of $NaBH_4$ (2.15 mmol, 1.3 eq) were then added and the reaction was stirred at 0°C for 35 minutes before addition of 0.12 mL of acetic acid. The reaction mixture was then stirred at room temperature for 30 min before addition of 30 mL of saturated aqueous $NaHCO_3$. The mixture was stirred for further 30 min at room temperature before extraction by 3 times 50 mL of ethyl acetate, the combined organic layers washed by 2 times 10 mL of saturated aqueous $NH_4Cl$, 10 mL of saturated aqueous NaCl, 15 mL of aqueous HCl 1M, 10 mL of saturated aqueous NaCl, dried over $Na_2SO_4$, filtered and the solvent evaporated. Purification by recrystallization from a mixture of diethyl ether and n-hexane yielded the pure triol.

Compound **1a**

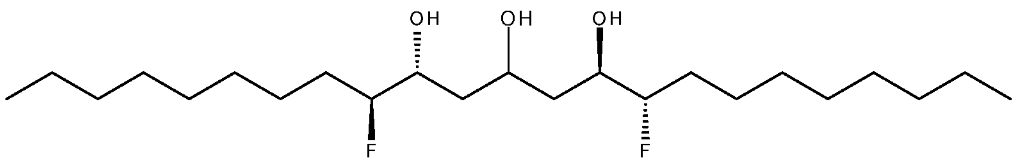

Compound **1a** was prepared according to procedure (B): 500 mg (1.2 mmol, 1 eq.) of the ketodiol **2a** were dissolved in 20 mL of dry THF and 1.5 mL of MeOH and cooled to 0°C under argon. 91.2 mg of $NaBH_4$ (2.4 mmol, 2 eq.) were then added and the reaction was stirred at 0°C for 2 hours before addition of 0.5 mL of acetic acid. The reaction mixture was then stirred at room temperature for 45 min before addition of 25 mL of saturated aqueous $NaHCO_3$. The mixture was stirred for further 30 min at room temperature before extraction by 3 times 30 mL of ethyl acetate, the combined organic layers were washed by 2 times with saturated aqueous $NH_4Cl$, 10 mL of saturated aqueous NaCl,

dried over $Na_2SO_4$, filtered and the solvent evaporated. The crude was filtered through a plug of silica ($Et_2O$) before purification by recrystallization from a mixture of ethyl acetate and pentane, to afford the triol **1a.**

421 mg (1,04 mmol). 86% yield. Rf = 0.24 (petroleum ether / ethyl acetate (7/3)).

The product was obtained in >99:1 *dr.*

$[\alpha]^{20}_D$ = -9.8° (THF, c = 1), 99/1 *dr*, >98% *ee.*

$^1H$ NMR (600 MHz, 333 K, $CD_3OD$): δ (ppm) = 0.89 (t, 2 CH3), 1.29-1.41 (m, 12 CH2), 1.51-1.77 (m, 4 CH2), 3.75-3.92 (m, 2 CH), 4.03-4.11 (m, CH), 4.19-4.26 (m, CH), 4.35-4.43 (m, CH).

$^{13}C$ NMR (151 MHz, 333 K, $CD_3OD$): δ (ppm) = 12.8 (s, CH3), 22.2 (s, CH2), 24.9 (s, CH2), 28.9 (s, CH2), 29.1 (s, CH2), 30.0-30.3 (m, CH2), 31.5 (s, CH2), 38.7 (d, J = 4 Hz, CH2), 39.3 (d, J = 4 Hz, CH2), 64.7 (d, J = 2 Hz, CH(OH)), 69.2 (d, J = 23 Hz, CH(OH)), 71.3 (d, J = 23 Hz, CH(OH)), 95.8 (d, J = 47 Hz, CH(F)), 96.9 (d, J = 47 Hz, CH(F)).

$^{19}F$ (NMR (376 MHz, $CD_3OD$): -191.9, -192.0.

HRMS ESI $[M+H]^+$ calculated for $C_{23}H_{47}F_2O_3^+$: 408.3415. Observed: 408.3427.

Determination of the stereoselectivity via Mosher ester formation:

To 7.8 mg of triol (0.015 mmol, 1 eq.) in 0.2 mL of dry dichloromethane were successively added 20.4 mg of (R)-Mosher acid (0.09 mmol, 6 eq.), 2.1 mg of DMAP (0.015 mmol, 1 eq.) and 19.6 mg of DCC (0.09 mmol, 6 eq.). The resulting mixture was stirred at room temperature for 3 hours. 4 mL of diethyl ether were then added, the organic layer was washed by 2 times with 3 mL of saturated aqueous $NaHCO_3$, 3 mL of saturated aqueous NaCl, 2 times with 3 mL of saturated aqueous $NH_4Cl$, 3 mL of saturated aqueous NaCl, dried over $Na_2SO_4$, filtered and the solvent evaporated. The crude product was directly analyzed by $^{19}F$ NMR to determine the stereoselectivity indicating a > 98:2 dr and > 98:2 er for the triol.

$^{19}F$ (NMR (376 MHz, $CDCl_3$): -71.14, -71.10, -70.81.

**Example 2:** Gelation tests

10 mg of triol were dissolved in 500 μL of the corresponding solvent in a small vial. The vial was capped before heating. The homogeneous solution was then cooled to room temperature. A sample was considered as a gel if when the vial was inverted, no solution dropped. The gel transition temperature (Tg) was determined at this concentration (CGC = 2 wt%) by heating gradually the gel and observing the optional gel scrambling by the inversion test. If a gel was formed (CGC = 2 wt%) further addition of small volumes of the corresponding solvent was performed and the heating/cooling cycle performed again. This protocol was repeated until the gel fails the inversion test to determine the CGC.

This method was relatively precise as confirmed by the quantitative results observed by Rheology. The triol gels are thermally reversible by heating up the gel to a temperature above Tg and cooling back again to room temperature.

As shown in Table 1, compound **1a** was tested in various solvents or oils. This compound was compared with compounds **3** (the side chains being an allyl moiety) and **4** (the side chains being a benzyl moiety). Compounds **3** and **4** were prepared according to procedures A then B, using the proper aldehyde.

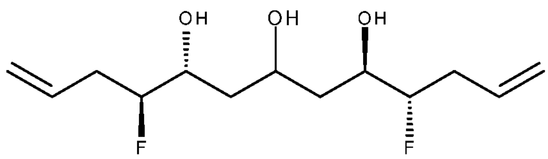

**3**

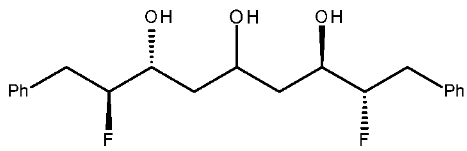

**4**

***Table 1***

| Solvent | CGC in wt/v%, Tg (°C) | | |
|---|---|---|---|
| | **1a** | **3** | **4** |
| EtOH | 2%, 30°C | NG | NG |
| $CH_3CN$ | 1%, 53°C | NG | NG |
| $CHCl_3$ | 2%, 31°C | NG | NG |
| $H_2O$ | Ins | Ins | Ins |

(continued)

| Solvent | CGC in wt/v%, Tg (°C) | | |
|---|---|---|---|
| | **1a** | **3** | **4** |
| Benzene | 0.5%, 28°C[d] | NG | NG |
| Toluene | 0.31%, 35°C[d] | NG | NG |
| Chloro benzene | 0.44%, 39°C[d] | NG | NG |
| *o*-xylene | 0.29%, 60°C[d] | NG | NG |
| *m*-xylene | 0.36%, 42°C | NG | NG |
| p-xylene | 1%, 53°C | NG | NG |
| Styrene | 0.57%, 50°C | - | - |
| Silicon oil | 2%, 35°C | - | - |
| Pump oil (paraffin) | 2%, 33°C | - | - |
| Higher order alkanes (dearomatized Petrole)[b] | 2%, 28°C | - | - |
| Higher order alkanes (+10% toluene) | 0.25%, 75°C | - | - |
| Higher order alkanes+35% toluene (Petrole) | 0.22%, 77°C[d] | NG | - |
| Diesel[c] | 0.66%, 50°C | - | - |

CGC = Critical Gel Concentration. Tg = Gel transition temperature.
NG = No gelation observed at 2 wt/v%. Ins = Insoluble.
a: Tg measured at 2 wt/v%.
b: drugstore bottle (Phebus brand from Charboneaux): hydroalkanes (C11-C14), iso-alcanes, n-alkanes, cyclics.
c: 75% saturated hydrocarbons (primarily paraffins including n, iso, and cycloparaffins), and 25% aromatic hydrocarbons (including naphthalenes and alkylbenzenes).
d: Translucent gel.

Low CGC were obtained with compound **1a**, from 0.22 wt/v% to 2 wt/v% in a wide range of solvents or oils. For a concentration of 2 wt/v%, Tg was comprised between 28°C and 77°C. No gelation at 2 wt/v% (or less) was observed for compounds **3** and **4**.

<u>**Example 3:** Anion sensing tests</u>

In a vial at room temperature, 10 mg of triol (with aliphatic chain R = n-$C_8H_{17}$) (0.025 mmol, 1 eq.) was added in 0.5 mL of toluene. The solubilization was performed by heating the vial. Then, the mixture was cooled to room temperature to obtain an organogel at 2 wt%. Tetrabutylammonium chloride (0.028 mmol, 1.1 eq.) was solubilized into 0.1 mL of toluene and was added into the vial containing the organogel. After 1 hour, a colorless liquid was obtained. This demonstrates the sensitivity of this organogel to chloride anions.

## Claims

**1.** A compound represented by formula (I):

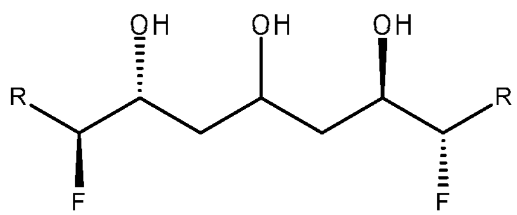

(I),

wherein R is a hydrocarbon aliphatic chain having from 6 to 26 carbon atoms,
and/or its
enantiomer,
wherein the expression "hydrocarbon aliphatic chain" refers to a linear or branched, cyclic or acyclic, non-aromatic hydrocarbon chain, optionally substituted, and optionally comprising at least one double bond and/or at least one triple bond.

**2.** The compound according to claim 1, wherein R is a hydrocarbon aliphatic chain having from 8 to 14 carbon atoms, preferably an octyl group.

**3.** Use of a compound as defined in claim 1 or 2, as an organogelator compound in a liquid sample comprising at least one oil and/or at least one organic solvent.

**4.** The use according to claim 3, for treating an oil spill.

**5.** An organogel obtained by contacting a compound as defined in claim 1 or 2, with a liquid sample comprising at least one oil and/or at least one organic solvent.

**6.** Use of an organogel as defined in claim 5 for anion sensing.

**7.** Use of an organogel as defined in claim 5 as a catalyst or in a catalytic system.

**8.** A composition comprising at least one compound as defined in claim 1 or 2, or at least one organogel as defined in claim 5.

**9.** The composition according to claim 8, which is selected from the group consisting of a lubricant, adhesive, molding, sealing, cosmetic composition or a coating composition such as a paint, a varnish or an ink.

**10.** A process for separating a hydrophobic phase from a mixture of hydrophobic and hydrophilic phases, comprising the steps of:

a) contacting at least one compound as defined in claim 1 or 2 with a mixture of hydrophobic and hydrophilic phases to obtain an organogel comprising the hydrophobic phase and a residue comprising the hydrophilic phase;
b) separating said organogel from said residue;

and optionally :

c) heating said organogel to a temperature allowing the deconstruction of said organogel into a hydrophobic phase and said at least one compound as defined in claim 1 or 2 ;
d) separating said hydrophobic phase and said at least one compound as defined in claim 1 or 2.

**11.** The process according to claim 10, wherein said hydrophobic phase is crude oil and said hydrophilic phase is water.

## Patentansprüche

**1.** Eine Verbindung, dargestellt mit Formel (I):

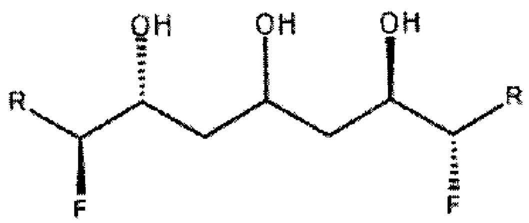

(I),

wobei R eine aliphatische Kohlenwasserstoffkette mit 6 bis 26 Kohlenstoffatomen und/oder ihr Enantiomer ist, wobei der Ausdruck "aliphatische Kohlenwasserstoffkette" eine lineare oder verzweigte, cyclische oder nicht-cyclische, nicht-aromatische Kohlenwasserstoffkette bezeichnet, die gegebenenfalls substituiert ist und gegebenenfalls wenigstens eine Doppelbindung und/oder wenigstens eine Dreifachbindung umfasst.

**2.** Die Verbindung gemäß Anspruch 1, wobei R eine aliphatische Kohlenwasserstoffkette mit 8 bis 14 Kohlenstoffatomen, vorzugsweise eine Octylgruppe ist.

**3.** Verwendung einer Verbindung, wie in Anspruch 1 oder 2 definiert, als eine Organogelator-Verbindung in einer Flüssigprobe, umfassend wenigstens ein Öl und/oder wenigstens ein organisches Lösemittel.

**4.** Die Verwendung gemäß Anspruch 3 zur Behandlung einer Ölverschmutzung.

**5.** Ein Organogel, erhalten durch Inkontaktbringen einer Verbindung, wie in Anspruch 1 oder 2 definiert, mit einer Flüssigprobe, umfassend wenigstens ein Öl und/oder wenigstens ein organisches Lösemittel.

**6.** Verwendung eines Organogels, wie in Anspruch 5 definiert, zur Aniondetektion.

**7.** Verwendung eines Organogels, wie in Anspruch 5 definiert, als einen Katalysator oder in einem katalytischen System.

**8.** Eine Zusammensetzung, umfassend wenigstens eine Verbindung, wie in Anspruch 1 oder 2 definiert, oder wenigstens ein Organogel, wie in Anspruch 5 definiert.

**9.** Die Zusammensetzung gemäß Anspruch 8, die aus der Gruppe ausgewählt ist, bestehend aus einer Schmiermittel-, Klebstoff-, Formmasse-, Dichtungsmasse-, Kosmetikzusammensetzung oder einer Beschichtungsmittelzusammensetzung wie einer Farbe, einem Lack oder einer Druckfarbe.

**10.** Ein Verfahren zur Trennung einer hydrophoben Phase von einem Gemisch aus hydrophober und hydrophiler Phase, umfassend die Schritte:

a) Inkontaktbringen wenigstens einer Verbindung, wie in Anspruch 1 oder 2 definiert, mit einem Gemisch aus hydrophober und hydrophiler Phase, um ein Organogel, umfassend die hydrophobe Phase, und einen Rückstand, umfassend die hydrophile Phase, zu erhalten;
b) Trennen besagten Organogels von besagtem Rückstand;
und gegebenenfalls
c) Erhitzen besagten Organogels auf eine Temperatur, die den Abbau besagten Organogels in eine hydrophobe Phase und besagte wenigstens eine Verbindung, wie in Anspruch 1 oder 2 definiert, erlaubt;
d) Trennen besagter hydrophober Phase und besagter wenigstens einer Verbindung, wie in Anspruch 1 oder 2 definiert.

**11.** Das Verfahren gemäß Anspruch 10, wobei besagte hydrophobe Phase Rohöl ist und besagte hydrophile Phase Wasser ist.

## Revendications

**1.** Composé représenté par la formule (I) :

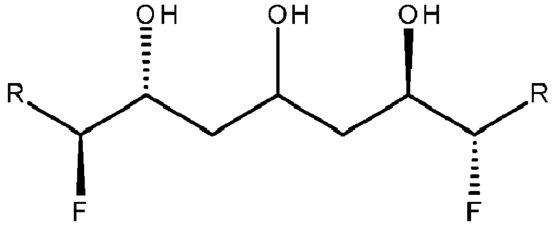

(I),

dans laquelle R est une chaîne aliphatique hydrocarbonée ayant de 6 à 26 atomes de carbone,
et/ou son énantiomère,
dans lequel l'expression "chaîne aliphatique hydrocarbonée" se réfère à une chaîne hydrocarbonée non aromatique, linéaire ou ramifiée, cyclique ou acyclique, éventuellement substituée, et comprenant éventuellement au moins une double liaison et/ou au moins une triple liaison.

**2.** Composé selon la revendication 1, dans lequel R est une chaîne aliphatique hydrocarbonée ayant de 8 à 14 atomes de carbone, de préférence un groupe octyle.

**3.** Utilisation d'un composé tel que défini dans la revendication 1 ou 2, en tant qu'organogélateur dans un échantillon de liquide comprenant au moins une huile et/ou au moins un solvant organique.

**4.** Utilisation selon la revendication 3, pour le traitement d'un déversement d'hydrocarbures.

**5.** Organogel obtenu par mise en contact d'un composé tel que défini dans la revendication 1 ou 2 avec un échantillon de liquide comprenant au moins une huile et/ou au moins un solvant organique.

**6.** Utilisation d'un organogel tel que défini dans la revendication 5 pour une détection d'anions.

**7.** Utilisation d'un organogel tel que défini dans la revendication 5 en tant que catalyseur ou dans un système catalytique.

**8.** Composition comprenant au moins un composé tel que défini dans la revendication 1 ou 2 ou au moins un organogel tel que défini dans la revendication 5.

**9.** Composition selon la revendication 8, qui est choisie dans l'ensemble constitué par un lubrifiant, un adhésif, un moulage, une étanchéité, une composition cosmétique ou une composition de revêtement telle qu'une peinture, un vernis ou une encre.

**10.** Procédé pour séparer une phase hydrophobe d'un mélange de phases hydrophobe et hydrophile, comprenant les étapes suivantes :

a) mise en contact d'au moins un composé tel que défini dans la revendication 1 ou 2 avec un mélange de phases hydrophobe et hydrophile pour que soit obtenu un organogel comprenant la phase hydrophobe et un résidu comprenant la phase hydrophile ;
b) séparation dudit organogel d'avec ledit résidu ;

et éventuellement :

c) chauffage dudit organogel à une température permettant la déconstruction dudit organogel en une phase hydrophobe et ledit au moins un composé tel que défini dans la revendication 1 ou 2 ;
d) séparation de ladite phase hydrophobe et dudit au moins un composé tel que défini dans la revendication 1 ou 2.

**11.** Procédé selon la revendication 10, dans lequel ladite phase hydrophobe est du pétrole brut et ladite phase hydrophile est de l'eau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 104892417
- US 20120108680 A
- US 20080113172 A
- US 20080113573 A


### Non-patent literature cited in the description


- **RAGHAVANPILLAI ; FRANCO.** *Appl. Sci.,* 2012, vol. 2, 175-191
- **QUINTARD ; RODRIGUEZ.** *ACS Catal.,* 2017, vol. 7, 5513-5517
- *ACS Catal.,* 2017, vol. 7, 5513